# EUROPEAN PATENT SPECIFICATION

(11) **EP 2 967 641 B1**
(45) Date of publication and mention of the grant of the patent: **25.04.2018**
(21) Application number: 14771124.6
(22) Date of filing: 14.03.2014
(51) Int. Cl.: A61B 17/322

(54) **MICROBLISTER SKIN GRAFTING**
MIKROBLISTER-HAUTTRANSPLANTATIONEN
GREFFE DE PEAU UTILISANT DES MICRO-VÉSICULES

(30) Priority: 15.03.2013 US 201313839518
(43) Date of publication of application: 20.01.2016
(73) Proprietor: KCI Licensing, Inc., San Antonio, TX 78265 (US)
(72) Inventor: SABIR, Sameer Ahmed, Cambridge, Massachusetts 02138 (US); ZIEGLER, Andrew, Arlington, Massachusetts 02476 (US)
(74) Representative: CMS Cameron McKenna Nabarro Olswang LLP
(86) International application number: PCT/US2014/027237
(87) International publication number: WO 2014/152346

(56) References cited:
- WO-A2-2007/034438
- WO-A2-2010/036788
- WO-A2-2012/019095
- SU-A1- 772 544
- US-A- 3 782 387
- US-A- 6 071 247
- US-A1- 2003 069 571
- US-A1- 2005 234 485
- US-A1- 2012 172 894
- US-A1- 2012 172 894
- US-A1- 2013 204 273

## Description

### Field of the Invention

The present invention generally relates to devices for generating and transferring skin grafts.

### Background

Skin is the largest organ of the human body, representing approximately 16% of a person's total body weight. Because it interfaces with the environment, skin has an important function in body defense, acting as an anatomical barrier from pathogens and other environmental substances. Skin also provides a semi-permeable barrier that prevents excessive fluid loss while ensuring that essential nutrients are not washed out of the body. Other functions of skin include insulation, temperature regulation, and sensation. Skin tissue may be subject to many forms of damage, including burns, trauma, disease, and depigmentation (e.g., vitiligo).

Skin grafts are often used to repair such skin damage. Skin grafting is a surgical procedure in which a section of skin is removed from one area of a person's body (autograft), removed from another human source (allograft), or removed from another animal (xenograft), and transplanted to a recipient site of a patient, such as a wound site.

As with any surgical procedure, skin grafting involves certain risks. Complications may include graft failure, rejection of the skin graft, infections at donor or recipient site, or autograft donor sites oozing fluid and blood as they heal. Certain of these complications (e.g., graft failure and rejection of the skin graft) may be mitigated by using an autograft instead of an allograft or a xenograft.

A problem encountered when using an autograft is that skin is taken from another area of a person's body to produce the graft, resulting in trauma and wound generation at the donor site. Generally, the size of the graft matches the size of the recipient site, and thus a large recipient site requires removal of a large section of skin from a donor site, leading to increased pain and discomfort and longer healing time. Additionally, as the size of the section of skin removed from the donor site increases, so does the possibility of infection.

Moreover, skin grafts are often difficult to obtain due to the tendency of the skin layer being cut to curl or fold over onto itself or the surgical instrument (e.g., dermatome), thereby comprising the integrity of the graft and making it unsuitable for use. This folding/curling tendency is particularly problematic the thinner the layer is that is being obtained, such as the epidermal layer.

While techniques have been developed for obtaining smaller micrografts that can be transferred onto a substrate for expansion prior to transplantation, such micrografts tend to clump together or can flip or fold during cutting, thereby comprising the integrity of the micrograft such that it will not properly grow on the substrate. As such, multiple cutting attempts are often necessary before a suitable, planar graft or micrograft is obtained, thereby producing multiple wound sites, leading to extreme discomfort, longer healing time, and a greater risk of infection.

Harvesting of a skin graft may be accomplished by many different techniques, and the technique used will depend on the type of graft to be harvested. A common technique to harvest a skin graft includes suction blistering. Suction blistering typically involves a heat source to warm the skin which facilitates blister formation.

The heat source of a suction blistering device can become overheated and burn out, causing inconsistent blister formation and potential harm to the patient. Thus, there is a need for a skin graft harvesting device with design features that prevent the device from overheating.

Current skin graft harvesting devices do not include means to monitor the development of suction blisters formation. Without such means, the device may be applied for overly long periods of time causing excessive discomfort or harm to the patient, or insufficient periods of time for causing blister formation. Thus, there is a need for a skin graft harvesting device with design features that allow the user to visually monitor the development of suction blisters on a patient.

A common technique for harvesting a skin graft includes creating one or more suction blisters, cutting the blister, and transferring the blister to a substrate, for example Tegaderm®. If the substrate is not sufficiently contacted with the suction blister, the blister won't transfer and will thus be unusable. As such, there is a need for a skin grafting device with design features that ensure full contact between the substrate and suction blister.

WO2012/019095 (which forms the basis for the preamble of claim 1) and US2012/172894 disclose devices for harvesting skin graft, providing a blister raising device integrated with a member for cutting the blister.

WO2010/036788 discloses methods and apparatuses for affecting an appearance of skin by harvesting small portions of tissue from a donor (first) site and applying them at a recipient (second) site.

US3782387 discloses an apparatus and method for obtaining skin grafts.

SU772544 discloses medical devices, namely surgical instruments for cutting skin grafts.

### Summary

The present invention provides a device for obtaining a skin graft, the device comprising: a harvester configured for placement on a target region of a patient's skin and further adapted to form a sealing engagement with a head that provides negative pressure to the target region such that the target region of skin is embraced within an evacuated chamber, the harvester further comprising: at least one alignment plate having a plurality of holes through which skin blisters can be raised in the presence of negative pressure; and a cutting plate having at least one cutting surface for cleaving skin blisters after they are formed within the chamber; wherein the cutting plate includes a plurality of holes suitable for concentric alignment with holes in the alignment plate in the first position to facilitate blister formation and a plurality of cutting surfaces suitable for cleaving blister in the second position, wherein the device further comprises an actuator for moving the cutting plate from the first position to the second position, characterized in that a single stroke of the actuator moves the cutting plate from the first position to the second position so as to cleave the skin blister and from the second position to the first position to at least partially retract the cutting plate.

A selection of optional features is set out in the dependent claims.

These and other aspects of the devices of the disclosure are described in the figures, description and claims that follow. While several improved design features have been individually described, such features are not mutually exclusive of each other. Any combination of design features disclosed herein can be used integrated into the devices of the disclosure. These design features and other aspects of the devices of the disclosure are described in the figures, description and claims that follow.

### Brief Description of the Drawings

Figure 1 provides a diagram showing the anatomy of skin.
Figure 2 panels A-C are schematics showing a device for generating and harvesting a plurality of micrografts. Panel A provides an exploded view of the device. Panel B provides a top view of the assembled device. Panel C provides a side view of the assembled device.
Figure 3 panels A-B is a drawing showing a device of the disclosure for raising a suction blister.
Figure 4 panels A-D show different devices of the disclosure for raising a suction blister.
Figure 5 panels A-B show schematics of head of a device according to the disclosure. Panel A provides a top view of the head. Panel B shows a side view of the head.
Figure 6 provides a diagram showing an external schematic of a device with a head coupled to a hollow body.
Figures 7A is a schematic depicting the components of an exemplary embodiment of a blister harvesting device according to disclosure;
Figure 7B is a schematic depicting the components of an exemplary embodiment of a blister generation module for coupling with the blister harvesting device of Figure 7A.
Figures 8A-8C are schematics depicting the assembly procedure of the components depicted in Figures 7A and 7B.
Figure 9 is a schematic depicting the components of an exemplary embodiment of a cutter assembly for use in the devices according to the disclosure.
Figure 10A is a schematic depicting an exemplary embodiment of a device according to the disclosure in a blister generation mode;
Figure 10B is a schematic depicting an exemplary embodiment of a device according to the disclosure in a blister harvesting mode.
Figures 11A-11C are schematics depicting the blister generation steps using the device mode depicted in Figure 10A.
Figure 12A-12C are schematics depicting the blister harvesting steps using the device mode depicted in Figure 10B.
Figure 13 is exploded schematic perspective view of another embodiment of a skin harvester according to the invention.
Figure 13A is a schematic perspective view of the harvester of Figure 13 as assembled.
Figure 13B is a schematic perspective sectional view of the harvester of Figure 13.
Figure 13C is an exploded schematic perspective view of the cutter and guide plates of the harvester of Figure 13.
Figure 13D is a top view of the cutter and guide plates of the harvester of Figure 13.
Figure 13E is a top view of a plate connector assembly of the harvester of Figure 13.
Figure 14A is a perspective view of the harvester of Figure 13 in an initial position to further illustrate the cutting mechanism.
Figure 14B is sectional side view of the cutter plate drive elements of the harvester in an initial position.
Figure 15A is a perspective view of the harvester of Figure 13 in a cocked position (handle up) to further illustrate the cutting mechanism.
Figure 15B is sectional side view of the cutter plate drive elements of the harvester in the cocked position.
Figure 16A is a perspective view of the harvester of Figure 13 in a mid-cut position to further illustrate the cutting mechanism.
Figure 16B is sectional side view of the cutter plate drive elements of the harvester in mid-cut position.
Figure 17A is a perspective view of the harvester of Figure 13 in transitional position (from cutting to retraction) to further illustrate the cutting mechanism.
Figure 17B is sectional side view of the cutter plate drive elements of the harvester in the cut-to-retract transitional position.
Figure 18A is a perspective view of the harvester of Figure 13 in a final (stroke completion) position to further illustrate the cutting mechanism.
Figure 18B is sectional side view of the cutter plate drive elements of the harvester in the final (partially retracted) position.

### Detailed Description

The present disclosure generally relates to a single device that can raise a blister (e.g., a suction blister) and cut the raised blister, i.e., a blister raising device integrated with a cutting member. Such devices are useful for harvesting skin grafts.

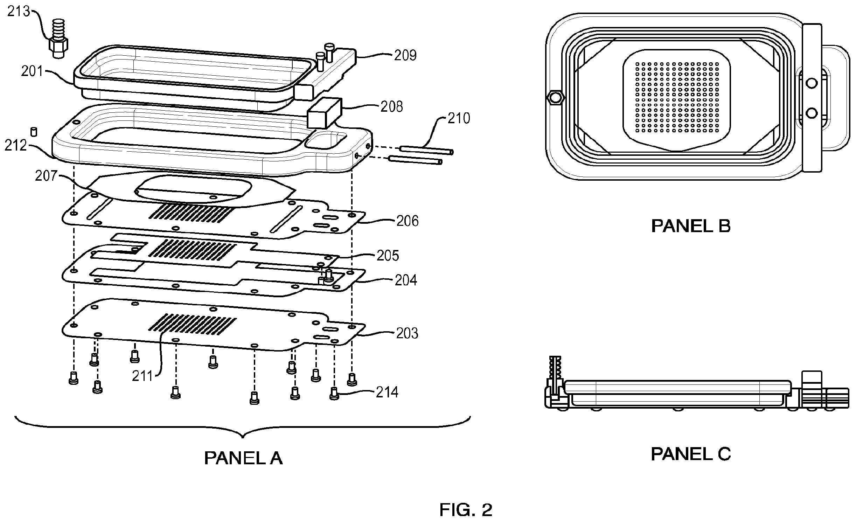

In certain embodiments, a device as shown in Figure 2 panels A-C is used to raise and cut a plurality of skin grafts. Device **200** includes a frame **201** and a lid **202.** Fitted into the frame is a bottom plate **203,** a cutter grid plate **204,** a cutter plate **205,** and a top plate **206.** The bottom plate **203,** the cutter plate **205,** and the top plate **206,** each include a hole array **211.** Once assembled, the hole array **211** of each of plates **203, 205,** and **206** are aligned. The size of the holes in the hole array will depend on the size of the graft needed, with larger holes being used to produce larger grafts. A first substrate **207** interacts with the top plate **206** and will receive the harvested grafts.

Device **200** further includes an actuation block **208,** actuation bar **209,** and actuation block guides **210.** Actuation components **208, 209,** and **210** control movement of the cutter plate **205.** The frame **201** includes a vacuum stop **212** and the lid **202** includes a suction hole barb **213.** Once assembled, the frame **201** and lid **202** are arranged such that the vacuum stop **212** and the suction hole barb **213** are aligned with each other (Figure 2 panel B). A vacuum source is then connected to the device **200** such that negative pressure can be generated within the device. The device **200** can be held together by clamp screws **214.** Device **200** may also include a heating element.

To produce and harvest the plurality of skin grafts, device **200** is placed on a donor site, such as an inner thigh of a patient. The vacuum source is turned on, producing negative pressure within device **200.** The negative pressure causes the skin to be pulled toward lid **202,** with a plurality of different portions of skin being pulled through each hole array **211** in each of plates **203, 205,** and **206.** Such action results in generation of many microblisters. Once the microblisters are raised, actuation components **208, 209,** and **210** are engaged to move cutter plate **205.** The movement of cutter plate **205** disrupts the alignment of the hole arrays **211** in each of plates **203, 205,** and **206,** and results in cutting of the microblisters. The cut microblisters are captured on the first substrate **207** that is above top plate **206.** In this manner, there is provided a spaced apart array of micrografts. The amount of negative pressure applied, the amount of time the vacuum is maintained, and/or the depth of the holes in plate **206** (i.e., the plate thickness) determine what type of graft will be harvested, e.g., epidermal graft, split thickness graft, or full thickness graft. Generally, each micrograft will have a lateral dimension of less than about 2 mm e.g., 100 to 2000 microns.

Another aspect of the disclosure provides a device for obtaining a single skin graft. Such devices of the disclosure include a hollow body having a distal end configured for placement on skin, a mechanism for raising a blister, and a cutter integrated in the body for cutting the blister produced on the skin.

A gauge for monitoring blister formation can be incorporated within one or more plates **203, 205,** and **206.** The gauge is preferably proximal to one or more holes of hole array **211** through which the blisters are formed. For example, the gauge can be located on the plate next to one or more holes of hole array **211,** or on an inner wall of one or more holes of hole array **211.** The gauge can be configured to provide minimum indicator of a sufficient height or dimension for a blister to be cut, and/or a maximum indicator of a sufficient blister dimension to avoid excessive patient discomfort by application of the device beyond a necessary period of time.

Each hole within the hole array has a depth substantially equal to the thickness of the plate. In certain embodiments, the gauge is a counterbore through one or more of the holes within hole array **211.** The counter bore serves as a marker to indicate to the user (e.g., clinician) when the blister has reached a dimension sufficient to be cut. The counterbore can be approximately one-half to three-quarters of the depth of the hole as measured from the bottom or distal-most surface of the plate (i.e., the surface closest to the skin). For example, if the plate is 7.62 mm (0.3 inches) thick, the counter bore is 3.81 mm (0.15 inches) to 5.715 mm (0.225 inches), as measured from the bottom or distal-most surface of the plate.

Alternatively, the gauge can be one or more calibration marks located proximal to or within one or more holes through which the blisters are raised. For example, the calibration marks can be one or more lines having a known length that are drawn, painted or etched onto the surface of the plate proximal to one or more of the holes. For example the one or more lines have a length of about 0.1 mm, about 0.2 mm, about 0.3 mm, about 0.4 mm, about 0.5 mm, about 1.0 mm, about 2.0 mm, about 3.0 mm, about 4.0 mm, about 5.0 mm, about 6.0 mm, about 7.0 mm, about 8.0 mm, about 9.0 mm, about 10.0 mm, about 11.0 mm, about 12.0 mm, about 13.0 mm, about 14.0 mm, about 15.0 mm, about 16.0 mm, about 17.0 mm, about 18.0 mm, about 19.0 mm, about 20.0 mm, about 21.0 mm, about 22.0 mm, about 23.0 mm, about 24.0 mm, or about 25.0 mm. Alternatively, at least two calibration marks can be drawn, painted or etched onto the surface of the plate proximal to one or more of the holes, and the distance between the at least two calibration marks can be a known length, for example about 0.1 mm, about 0.2 mm, about 0.3 mm, about 0.4 mm, about 0.5 mm, about 1.0 mm, about 2.0 mm, about 3.0 mm, about 4.0 mm, about 5.0 mm, about 6.0 mm, about 7.0 mm, about 8.0 mm, about 9.0 mm, about 10.0 mm, about 11.0 mm, about 12.0 mm, about 13.0 mm, about 14.0 mm, about 15.0 mm, about 16.0 mm, about 17.0 mm, about 18.0 mm, about 19.0 mm, about 20.0 mm, about 21.0 mm, about 22.0 mm, about 23.0 mm, about 24.0 mm, or about 25.0 mm. As the blister is formed, the lateral dimension of the blister can be compared to the one or more calibration marks to gauge when the blister is ready to be cut.

In yet another embodiment, the calibration marks may be one or more markings that are drawn, painted or etched onto the inner wall of one or more holes within hole array 211 of the plates. Such markings can indicate a minimum depth within the hole that is sufficient for a blister to be cut, and a maximum level for sufficient blister formation to avoid excessive patient discomfort by application of the device beyond a necessary period of time.

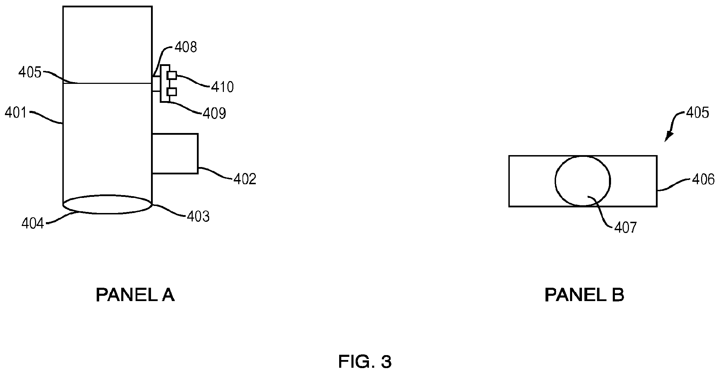

In certain embodiments, a device as shown in Figure 3 panel A is used to obtain a skin graft. Device **400** includes a hollow body **401** and a mechanism for raising a blister **402.** Hollow body **401** includes a distal end **403** that is configured for placement on the skin. Such a distal end may include an orifice plate **404.** Orifice plate **404** determines the size and the shape of the blister or blisters that will be raised. Orifice plate **404** may be any shape or size and will depend on the blister to be raised. Generally, the diameter or lateral dimension of the blister may be from about 6 mm to about 12 mm, although larger or smaller blister sizes may be used.

The mechanism for raising a blister may be a vacuum component, a heating component, or a combination thereof. An exemplary heating component is a light source. In a particular embodiment, mechanism **402** is a combination of a vacuum component and a heating component.

The hollow body **401** further includes a cutter **405,** which includes cutter plate **406** and a hole **407** (Figure 3 panel B). Device **400** further includes an actuation block **408,** actuation bar **409,** and actuation block guides **410.** Actuation components **408, 409,** and **410** control movement of the cutter **405.**

Blister formation is accomplished by attaching the distal end **403** of hollow body **401** to donor site of a patient, such as an inner thigh of a patient. Hook and loop fastener straps may be used to keep the device in place. The heating component of blister raising mechanism **402** provides a slight warming of orifice plate **404,** which is in direct contact with the patient's skin surface. The application of a moderate negative pressure to the chamber interior from the vacuum component of blister raising mechanism **402,** results in the patient's skin being gently drawn through the opening in orifice plate **404.** The result is a blister or blisters, approximately the size of the opening in orifice plate **404.** The produced blister may be fluid-filled or may not contain any fluid, i.e., a blister having air within. The skin and blister area is generally not damaged and patient discomfort is minimal.

The cutter **405** is positioned in hollow body **401** such that upon raising the blister, at least a portion of the blister protrudes through hole **407** in cutter plate **406.** The actuation components **408, 409,** and **410** are engaged to move cutter plate **406.** The movement of cutter plate **406** disrupts the alignment of hole **407** with the other components of device **400,** and results in cutting of the raised blister.

Preferably, the blister raising mechanism **402** is capable of emitting heat ranging between about 100 °C to about 750 °C (e.g., about 500 °C). In certain aspects, the blister raising mechanism **402** emits electromagnetic radiation having a wavelength ranging between about 10 nm and about 3000 nm. In certain aspects, electromagnetic radiation emitted from blister raising mechanism **402** is reflected off one or more of the surfaces within the device, back to mechanism **402,** causing it to overheat and burnout. To prevent overheating of mechanism **402,** at least one plate of plates **203, 205,** and **206** and/or orifice plate(s) **404** can include at least one surface configured for attenuating the reflection of electromagnetic radiation emitted from mechanism **402.** Preferably such surface is the surface facing mechanism **402** when the device is fully assembled.

For example, at least one surface of one or more of plate members **206, 205, 203** and/or orifice plate **404** can be coated with a material that substantially attenuates reflection of the electromagnetic radiation (e.g., by absorbing) emitted from mechanism **402.** Suitable materials include, for example, a thermoplastic polymer coating. In a particular embodiment, the thermoplastic polymer is a fluoropolymer such as polytetrafluoroethylene. Preferably, the coating material is a dark color such as a substantially black, brown, blue or purple color.

Alternatively, one or more of plate members **206, 205, 203** and/or orifice plate **404** can be anodized, electroplated or painted a dark color such as black, brown, blue or purple to attenuate the reflection (e.g., absorb) of electromagnetic radiation emitted from mechanism **402.**

In yet another embodiment one or more of plate members **206, 205, 203** and/or orifice plate **404** can be abraded, scuffed, brushed, or the like, to minimize or remove a glossy or shiny surface appearance in order to attenuate reflection of electromagnetic radiation from mechanism **402.**

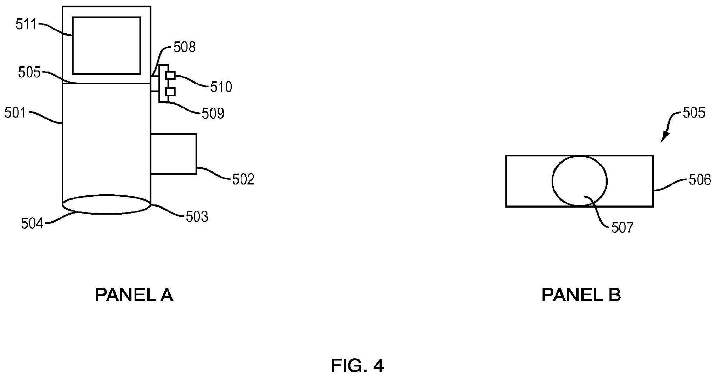

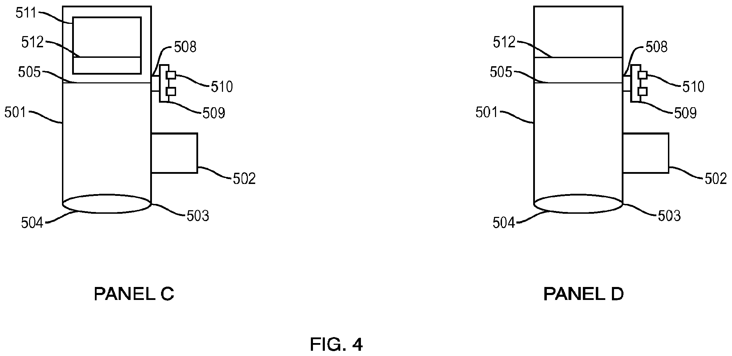

Figure 4 panel A shows a device **500** that further includes a chamber **511** for capturing the cut blister. Chamber **511** is positioned in hollow body **501** and above cutter **505.** Chamber **511** may be removable from device **500.** Chamber **511** may include multiple configurations. For example, chamber **511** may include a retractable bottom. The bottom is in an open position when chamber **511** is inserted into hollow body **501.** In the open position, chamber **511** is able to receive the cut blister. Once the cut blister is in chamber **511,** the bottom of the chamber is closed, capturing the blister in chamber **511.** Chamber **511** may then be removed from device **500.**

In another embodiment, chamber **511** includes a substrate **512** (Figure 4 panel C). In this embodiment, device **500** is configured such that substrate **512** is positioned in chamber **511** so that upon raising the blister, a portion of the blister contacts the substrate and becomes attached to the substrate. Cutter **505** then cuts the blister, and the cut blister becomes attached to the substrate **512** in chamber **511.** Chamber **511** is then removed from device **500,** and substrate **512** may be removed from chamber **511.** In other devices, a vacuum, instead of a substrate, is used to hold the cut blister within the chamber.

In certain embodiments, device **500** does not use a chamber, rather a substrate 512 is directly integrated with device **500** in order to capture the cut blister (Figure 4, panel D). Once captured, substrate **512** having an attached cut blister may be removed from device **500.**

In certain embodiments, the device **500** includes a substrate compression mechanism for pressing the substrate against the blister to ensure that the entire blister surface contacts the substrate **512.** Full contact between the entire blister surface and the substrate ensures transfer of the blister onto the substrate when the blisters are cut. In certain embodiments, the compression member is movably coupled to an exterior surface of the hollow body and actuated by an actuation member coupled to the compression member.

The compression member can be a plate having approximately the same size and shape as substrate **512.** The plate can be coupled to the hollow body via a hinged mechanism or axle member and is actuated by an extension arm or handle fixedly attached to the plate. The extension arm/handle is engineered to apply at least about 2x, at least about 3x, at least about 4x, at least about 5x, at least about 6x, at least about 7x, at least about 8x, at least about 9x, at least about 10x, at least about 15x, at least about 20x, at least about 25x, at least about 30x, at least about 35x, at least about 40x, at least about 50x, at least about 75x, at least about 100x the pressure applied to the extension arm/handle onto the plate.

Alternatively, the compression mechanism can be a cylindrical roller disposed about an actuation arm that defines a longitudinal axis. Movement of the arm in a lateral direction translates into rotational movement of the cylinder about the longitudinal axis of the arm, such that the cylinder is rolled across the surface of the substrate **512** to press the substrate against the blisters.

The compression member and/or actuation member are preferably reusable. Alternatively, the compression member and/or actuation member are made of a disposable material. Materials for the construction of the compression plate or cylinder can be any substantially solid material such as an elemental metal, a metal alloy, a glass, a crystal, or a polymer. In certain embodiments, the compression member and/or actuation member are made of titanium or stainless steel.

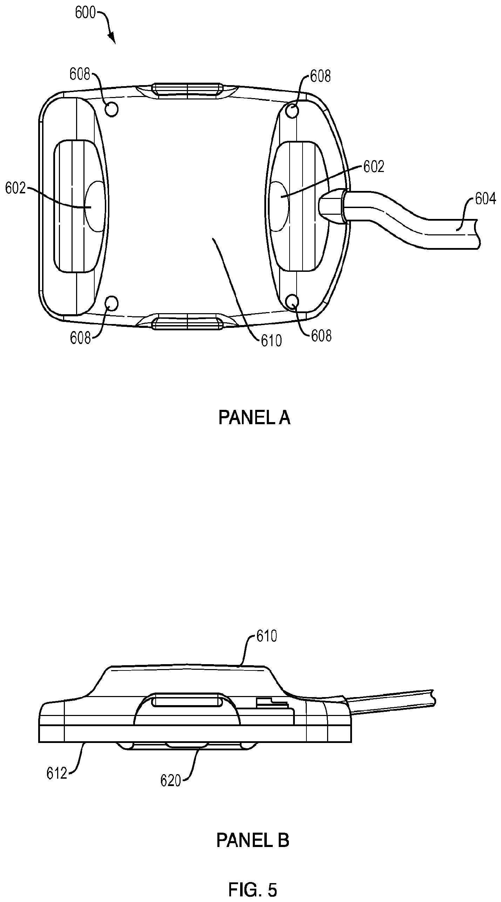

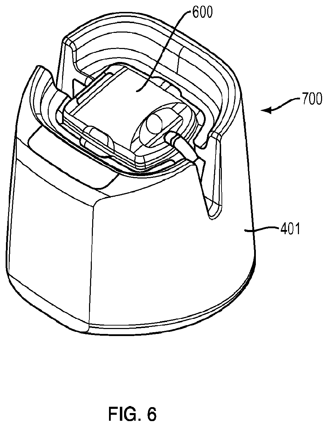

In certain embodiments, the devices include a head portion that can be removably coupled with the hollow body **401** of the device. Figure 5 shows an exemplary embodiment of a removable head **600** that includes a blister raising mechanism **402** (e.g., a heating element) for raising a suction blister. The head **600** includes a topmost, proximal portion **610,** and a distal portion **620** that couples with the hollow body of the device. The head **600** is coupled to the hollow body **401** via holes **608.** After attachment of head **600** to the hollow body **401,** a vacuum source can be attached to suction tubing **604** to generate negative pressure within the hollow body of the device. Figure 6 shows head **600** coupled to hollow body **401** (collectively 700).

In certain embodiments, the head device includes one or more viewing windows **602.** The viewing windows are located to provide optimal viewing of blister formation within the hollow body of the device. As shown in Figure 5, a plurality of viewing windows **602** can be integrated within the head **600** to allow for alternative views of blister formation, or allow more than one user to monitor the development of the blisters. In certain embodiments, an ocular shield circumscribes the viewing lens such then when the user is viewing blister formation, the shield attenuates entrance of ambient light into the viewing lens.

The viewing window **602** can be made of any transparent material. In preferred embodiments, the viewing window **602** is comprised of optical quality material, for example an optical polymer, an optical glass, or an optical crystal. Such materials can further include one or more of an anti-fogging material, an anti-scratch coating, or an anti-glare coating, located on either the or both the interior surface, the exterior surface, or both.

In certain embodiments, the viewing window is made of a heat resistant optical polymer, optical glass, or optical crystal to prevent warping or distortion from the heating element of the blister raising mechanism **402** within the head **600.**

At least a portion of the viewing window **602** can further include a magnification lens to facilitate viewing of the blisters during formation. The magnification power of the lens can be at least about 2x, at least about 3x, at least about 4x, at least about 5x, at least about 6x, at least about 7x, at least about 8x, at least about 9x, at least about 10x, at least about 15x, at least about 20x, at least about 25x, at least about 30x, at least about 35x, at least about 40x, at least about 50x, at least about 75x, at least about 100x.

In still other embodiments, the viewing window **602** can include one or more calibration marks etched or painted on the viewing window **602** for monitoring blister formation. Where the viewing window **602** includes a magnification lens, the calibration marks can be calibrated to the magnification power of the lens to approximate the actual dimensions of the forming blister, such as the actual height, the actual diameter, or both. When the desired blister size is formed as gauged by the calibration marks, the blisters are cut.

As previously described, the head **600** can include a mechanism for raising a blister **402.** Such mechanism typically includes a heating element, such as nichrome wire, and is located in the topmost, proximal portion **610** of head **600**

In certain embodiments head **600,** includes a transparent or a translucent surface **620** forming the distal side **612** of the head **600** (i.e., distal to the heating element). The transparent or translucent surface is made of a material that facilitates the transmission of electromagnetic radiation emitted from the heating element within head **600** to one or more plate members incorporated within the hollow body, thereby warming the plate members and subsequently the skin surface.

In certain aspects, the transparent or translucent surface is made of material that allows light having a wavelength between about 10 nanometers to about 3000 nanometers to be transmitted through the surface. Suitable materials for transmission of light within such rangeincludes, for example, crystalline materials such as sapphire, quartz, silicon, garnet, sillenite, fused silica, fused quartz, titanium dioxide, zinc selenide, calcium fluoride, barium fluoride, zinc sulphide, caesium iodide, germanium, thallium bromo-iodide, lithium fluoride, magnesium fluoride, potassium bromide, sodium chloride, or strontium fluoride. The crystalline material can be polarized.

Other suitable materials include glass such as silica glass, fluoride glass, aluminosilicate glass, phosphate glass, borate glass, chalcogenide glass, or polymer glass. The glass can be polarized.

In certain aspects, the head **600** includes two transparent or translucent surfaces **620** forming the distal side **612** of head **600.** The two plates surfaces are in a stacked configuration with an airspace in between them. The airspace between the transparent or translucent surfaces is about 0.1 mm, about 0.2 mm, about 0.3 mm, about 0.4 mm, about 0.5 mm, about 0.6 mm, about 0.7 mm, about 0.8 mm, about 0.9 mm, about 1.0 mm, about 2.0 mm, about 3.0 mm, about 4.0 mm, about 5.0 mm, about 6.0 mm, about 7.0 mm, about 8.0 mm, about 9.0 mm, about 10.0 mm, about 11.0 mm, about 12.0 mm, about 13.0 mm, about 14.0 mm, about 15.0 mm, about 16.0 mm, about 17.0 mm, about 18.0 mm, about 19.0 mm, about 20.0 mm, about 21.0 mm, about 22.0 mm, about 23.0 mm, about 24.0 mm, or about 25.0 mm.

The two transparent or translucent surfaces can be the same materials, or different materials. For example, the two surfaces can both be made of a glass or crystalline material. Alternatively, one of the surfaces is a glass material, while the other surface is a crystalline material.

In another aspect, the disclosure relates to an integrated device for generating micrografts and transferring micrografts. More specifically, the disclosure relates to a device for generating substantially planar micrografts and for preparing a surgical dressing to facilitate presentation of the micrografts to a patient in need thereof. The device of the disclosure can be used to prepare any type of skin graft, such as an epidermal skin graft, a split thickness graft, or a full thickness graft. However, the device of the disclosure is particularly well suited for preparing skin grafts including only or substantially only the epidermal layer of skin. The device of the disclosure can be used for autografts, allografts, or xenografts. In preferred embodiments, the grafts are autografts.

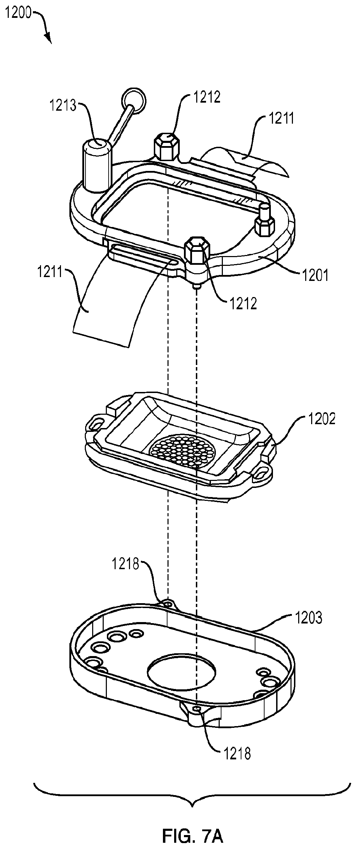

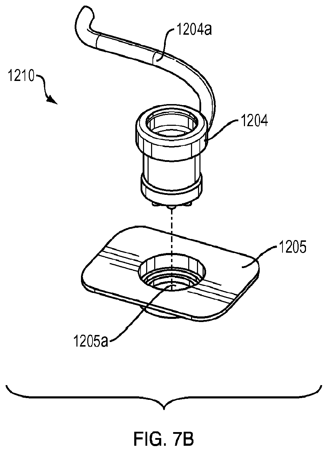

Referring now to **FIGS. 7A** and **7B****,** device **1200** includes a top housing **1201,** a cutter assembly **1202** and a base housing **1203.** The top housing includes a rotatable handle **1213** that is coupled to the cutter assembly **1202.** The top housing further includes a strap **1211** for coupling the device **1200** (once assembled) against a skin surface. The strap may be adjustable in size, or may be a fixed size. The top housing **1201** is configured to removably receive a blister generation module **1210** that includes a blister generation device **1204** and an adaptor plate **1205 (****FIG. 2B****).**

**FIGS 3A-3C** depict the assembly of device **1200.** As shown in **FIG. 3A****,** cutter assembly **1202** is inserted into top housing **1201.** Top housing **1201** is then coupled to base housing **1203** via one or more threaded screws **1212** that are received by a corresponding threaded holes **1218** in base housing **1203,** such that cutter assembly **1202** is disposed in between top housing **1201** and bottom housing **1203 (****FIG. 3B****).** As shown in **FIG. 3C****,** the blister generation module **1210** is then inserted into top housing **1201.** In certain embodiments, the bottom of adaptor plate **1205** that interfaces with top housing **1201** includes a gasket around the bottom perimeter of the plate **1205** to create an airtight seal between adaptor plate **1205** and top housing **1201** when coupled together. The blister generation device **1204** of the blister generation module **1210** is coupled to an opening **1205a** within adaptor plate **1205.** In certain embodiments, a gasket is disposed within opening **1205a** to form an airtight seal between blister generation device **1204** and adaptor plate **1205** when coupled together.

Referring now to **FIG. 4****,** the cutter assembly **1202** of device **1200** is shown. The cutter assembly **1202** includes a bottom plate **1202a,** a middle plate **1202b,** and a top plate **1202c,** each of which include an array of openings **1214** (e.g., holes or slots) (sometimes referred to herein as hole array **1214).** One or more openings of the hole array **1214** in the bottom **1202a,** middle **1202b** and/or top **1202c** plates define a cutting edge or surface **1215.** Preferably one or more openings in the hole array **1214** of at least the middle plate **1202b** define a cutting edge or surface **1215 (****FIG. 4****).** The three plates are assembled in a stacked configuration with the middle plate **1202b** being coupled to the bottom plate **1202a,** and the top plate **1202c** being coupled to the middle plate **1202b.** One or more of plates **1202a, 1202b** and **1202c** are configured to be movable in a lateral direction relative to each other. For example, the middle plate **1202b** may be laterally movable relative to the bottom plate **1202a,** the top plate **1202c,** or both. The top plate **1202c** may be movable relative to the middle plate **1202b,** the bottom plate **1202a,** or both. In certain embodiments, the one of more of plates **1202a, 1202b** and **1202c** are configured to laterally move within a fixed distance relative to each other.

The middle plate **1202b** and/or top plate **1202c** can be coupled to their respective plates in the stacked configuration via at least one frangible section which serves to keep the plates in alignment until a lateral force is applied to the middle **1202b** and/or top **1202c** plate, which breaks the frangible section(s) and allows lateral movement of the plates relative to each other. In a particular embodiment, at least the middle plate **1202b** is coupled to the bottom plate **1202a** via at least one frangible section. The at least one frangible section is configured to break when a lateral force is applied to the middle plate **1202b,** allowing the middle plate **1202b** to move in a lateral direction relative to the bottom plate **1202a,** the top plate **1202c,** or both. Preferably, middle plate **1202b** is configured to laterally move within a fixed distance relative to the bottom plate **1202a** and/or top plate **1202c.** In a particular embodiment, the middle plate **1202b** includes one or more grooves or channels **1216** that are configured to receive a pin **1217** vertically extending from bottom plate **1202a.** Pin **1217** is received at one end of channel **1216** when the frangible section is intact, and laterally slides within channel **1216** to the opposite end when the frangible section is broken, such that the lateral movement of plate **1202b** relative to plate **1202a** and/or **1202c** is fixed by the movement of pin **1217** within channel **1216.**

One or more coupling members can be disposed between the plates to form the frangible sections, as described in further detail below. The one or more coupling members are disposed between the openings within hole array **1214.** Alternatively, the one or more coupling members are disposed between the plates outside of hole array **1214.** The frangible coupling of the plate members to each other can be accomplished using a mechanical stamping technique, a mechanical punch technique, spot welding, photo etching, an epoxy, an adhesive, mechanical compression, a snap-fit assembly, a tongue and groove assembly, a post and bar assembly, a frangible pin, or any combination thereof.

In certain embodiments, the middle plate **1202b** and/or top plate **1202c** can be coupled to their respective plates in the stacked configuration via at least one elastic member or spring member which serves to keep the plates in alignment until a lateral force is applied to the middle **1202b** and/or top **1202c** plate, which allows the elastic/spring section(s) to flex and allows lateral movement of the plates relative to each other. Upon removal of the lateral force, the elastic/spring sections relax, which allows the plates to return to their original positions such that the hole arrays **1214** between the plates are once again in concentric alignment. The one or more elastic coupling members or spring members can be disposed between the openings within hole array **1214.** Alternatively, the one or more elastic coupling members or spring members can be disposed between the plates outside of hole array **1214.**

Preferably, the hole arrays **1214** of the bottom **1202a,** middle **1202b** and top **1202c** plates include holes that are substantially similar in size and substantially cylindrical in shape. The size of the holes in each hole array **1214** will depend on the size of the graft needed, with larger holes being used in each plate to produce larger grafts. In certain embodiments, the holes in the hole array 1214 range between 1 mm and 12 mm in diameter, or any specific value in between. For example, the diameter of the holes in the hole array **1214** of one or more of plates **1202a, 1202b** and **1202c** can be 1 mm, 1.5 mm, 2 mm, 2.5 mm, 3 mm, 3.5 mm, 4 mm, 4.5 mm, 5 mm, 5.5 mm, 6 mm, 6.5 mm, 7 mm, 7.5 mm, 8 mm, 8.5 mm, 9 mm, 9.5 mm, 10 mm, 10.5 mm, 11 mm, 11.5 mm or 12 mm. In certain embodiments, the holes in hole array **1214** vary in size and/or shape between the bottom plate **1202a,** middle plate **1202b** and/or top plate **1202c.** Once plates 1202a, **1202b** and **1202c** of cutter assembly **1202** are assembled (i.e., in the stacked configuration), the hole array **1214** of each of plates **1202a, 1202b,** and **1202c** are aligned. In a particular embodiment, hole arrays **1214** of plates **1202a, 1202b,** and **1202c** are concentrically aligned.

The device **1200** has two modes of operation: 1) a blister generation mode **(****FIG. 5A****);** and 2) a blister harvesting mode **(****FIG. 5B****).** As shown in **FIG. 5A****,** the blister generation mode includes the assembly with the blister generation module **1210.** The blister generation module **1210** is removed from the device assembly for blister harvesting mode **(****FIG. 5B****).** To produce and harvest a plurality of substantially planar micrografts, device **1200** in the blister generation mode (i.e., with blister generation module **1210,** as shown in FIG. 5A), is placed on a donor site **1220** such as an inner thigh of a patient (**FIG**. **6A**). Strap **1211** is used to keep the device **1200** in place against the skin surface of donor site **1220.** The blister generation device **1204** is activated by turning/cranking handle **1204a** of blister generation device **1204.** The blister generation device **1204** utilizes a vacuum component, a heating component, or a combination thereof, for raising skin blisters. An exemplary heating component is a light source. In a particular embodiment, mechanism is a combination of a vacuum component and a heating component.

In certain embodiments, the blister generation device **1204** is a suction blister device for suction blister grafting. Suction blister grafting involves raising a skin blister, and then cutting off the raised blister. An exemplary suction blister grafting technique is shown in Awad, (Dermatol Surg, 34(9):1186-1193, 2008). This article also shows various devices used to form suction blisters. A suction blister device is also described in Kennedy et al. (U.S. 6,071,247). An exemplary device is the commercially available Negative Pressure Cutaneous Suction System from Electronic Diversities (Finksburg, MD).

A device for raising a suction blister typically operates by use of suction chambers that are attached to a patient's skin. An instrument typically contains a power source, a vacuum pump, temperature controls and all related controls to operate multiple suction chambers. The suction chambers are connected to the console by a flexible connection. Each of the chambers is controlled by a preset temperature control to provide an optimal skin warming temperature. Both chambers share an adjustable common vacuum source that affects all chambers equally.

The chamber heating system provides a slight warming of an orifice plate of the device, which is in direct contact with the patient's skin surface. The negative pressure chamber is fabricated of mostly plastic components, with two removable threaded caps. The upper cap is fitted with a clear viewing lens so that the actual blister formation can be observed. The opposite end of the chamber is fitted with a removable orifice plate that is placed on the patient's skin. Since this plate is simply threaded onto the chamber end, multiple plates with different opening patterns can be interchanged as desired.

The interior of the device is warmed and illuminated by an array of low voltage incandescent lamps. This lamp array is controlled from the instrument console temperature controller, cycling as needed, to maintain the set point temperature. The heat from these lamps is radiated and conducted to the orifice plate, which then warms the patient's skin. The chamber is connected to the console via a composite vacuum and low voltage electrical system. Quick connections are used for the vacuum and electrical system to facilitate removal and storage.

The Negative Pressure Instrument console is a self-contained fan cooled unit which is designed to operate on 120 VAC 60 Hz power. Vacuum is supplied by an industrial quality diaphragm type vacuum pump, capable of a typical vacuum of 20 in Hg (0-65 kpa) at 0 CFM. An analog controller that is preset to 40 °C provides the temperature control for each suction chamber. This provides accurate control of the orifice plate temperature. The instrument console has internal adjustments that allow the user to recalibrate the temperature setting if desired. Other temperatures can be preset if desired. The front panel includes a vacuum gauge and vacuum bleeder adjustment to regulate the vacuum to both chambers. The console front panel also contains the connections for the chamber assemblies.

The application of a moderate negative pressure from the blister generation device **1204** causes the patients skin to be gently drawn through the concentrically aligned hole arrays **1214** of plates **1202a, 1202b** and **1202c** in cutter assembly **1202** (**FIG**. **6B**). Such action results in generation of a plurality of raised microblisters **1221,** particularly epidermal microblisters. The blisters **1221** may or may not be fluid-filled. The plurality of suction blisters **1221** generated are of uniform size, approximately the size of the openings/holes in the hole arrays **1214** of the three plates of cutter assembly **1202,** and are uniformly spaced in accordance with the configuration of the holes in hole array **1214,** such that a plurality of substantially planar microblisters **1221** are generated. The skin and blister area is generally not damaged and patient discomfort is minimal.

Once the substantially planar microblisters **1221** are raised/generated the device is converted into the blister harvesting mode by removing the blister generation module **1210** from the top housing **1201,** thereby exposing the hole array **1214** in the top plate **1202c** of cutter assembly **1202.** At least a portion of the raised microblisters **1221** protrude through the top of the hole array **1214,** as shown in **FIGS. 6B** and **6C****.** A substrate **1219** is applied to the surface of hole array **1214,** as shown in **FIGS. 5B** and **7A****,** such that the substrate **1219** is in direct contact with the raised blisters **1221.**

To cut the raised blisters **1221,** handle **1213** is rotated in a clockwise or counterclockwise direction (**FIG**. **7B**). Handle **1213** is coupled to the middle plate **1202b** of cutter assembly **1202** in a configuration that translates the rotational movement of the handle **1213** into lateral movement of middle plate **1202b.** The lateral force applied to middle plate **1202b** by handle **1213** causes middle plate **1202b** to move in a lateral direction relative to bottom plate **1202a** and/or top plate **1202c,** thereby disrupting the alignment of the hole arrays **1214** between plates **2012a, 2012b** and **1202c.** The lateral displacement of the hole array **1214** of middle plate **1202b** causes the cutting surface **1215** defined by one or more holes in the hole array **1214** to cut the raised blisters **1221.** As the raised blisters **1221** are cut, they are simultaneously transferred/retained on substrate **1219** in the same configuration as generated within hole array **1214,** resulting in a substrate containing a plurality of micrografts that are uniformly spaced and oriented on the substrate **1219** (i.e., a substrate containing a plurality of substantially planar micrografts).

Certain embodiments of device **1200** integrate consumable/single-use components (e.g., substrate **1219** and/or cutter assembly **1202)** and re-usable, sterilizable or cleaned components (e.g., top housing **1201,** base housing **1203** and blister generation module **1210**), thereby providing a reliable system that is easy to maintain. All components of device **1200** that come into contact with the donor and/or recipient tissue (both single-use and reusable components) must be sterile/sterilized to reduce the risk of infection.

In certain embodiments, substrate **1219** includes an adhesive on one side that facilitates attachment of the blisters to the substrate. The substrate material may have intrinsic adhesive properties, or alternatively, a side of the substrate may be treated with an adhesive material, e.g., an adhesive spray such as LEUKOSPRAY (Beiersdoerf GmbH, Germany). The substrate may be a deformable non-resilient material. A deformable non-resilient material refers to a material that may be manipulated, e.g., stretched or expanded, from a first configuration to a second configuration, and once in the second configuration, there is no residual stress on the substrate. Such materials may be stretched to an expanded configuration without returning to their original size. Such deformable non-resilient materials tend to be soft, stiff or both soft and stiff. Softness is measured on the durometer scale. An example of such a material is a soft polyurethane. A soft polyurethane is produced is as follows. Polyurethanes in general usually have soft and hard segments. The hard segments are due to the presence of phenyl bridges. In a soft polyurethane, the phenyl bridge is switched out for an aliphatic, which is more flexible as its 6 carbon ring has no double bonds. Therefore, all the segments are soft. On the Durometer Scale, a soft polyethylene is rated about Shore 80A. Other materials suitable for use with the device **1200** of the invention include low density polyethylene, linear low density polyethylene, polyester copolymers, polyamide copolymers, and certain silicones. In a particular embodiment, the substrate **1219** is Tegaderm™.

Ultimately, the substrate containing the plurality of uniformly spaced and oriented (i.e., substantially planar) micrografts is applied to a recipient of site of a patient. Prior to applying the grafts to the recipient site, the site is prepared to receive the grafts using any technique known in the art. Necrotic, fibrotic or avascular tissue should be removed. The technique used to prepare the site will depend on damage to the recipient site. For example, epidermal tissue, if present at the recipient site, can be removed to prepare the area for receiving the micrografts. Burned or ulcerated sites may not need removal of epidermal tissue, although some cleaning of the site or other preparation of the site may be performed. Wounds should be debrided and then allowed to granulate for several days prior to applying the graft. Most of the granulation tissue should be removed since it has a tendency to harbor bacteria. Applying silver sulfadiazine to the wound for 10 days prior to grafting reduces the bacterial count greatly.

The size of the area at the recipient site can be about the same size as the area of the substrate having micrografts adhered thereto. This size generally will be greater than the area of the original graft tissue that was removed from the donor site to form the micrografts. The depigmented or damaged skin can be dermabraded with sandpaper or another rough material. Alternatively, the epidermal tissue can be removed from the recipient site by forming one or more blisters over the area to be treated, e.g., a suction blister or a freezing blister, and the raised epidermal blister tissue can then be removed by cutting or another procedure.

The substrate having the substantially planar micrografts can be placed over the area to be treated to form a dressing. A portion of the substrate having the micrografts can be positioned over the area to be repaired, e.g., the area from which the epidermal tissue has been abraded or removed for repigmentation. The substrate can be fixed in place over the treatment area, e.g., using tape or the like. The substrate can be removed after sufficient time has elapsed to allow attachment and growth of the micrografts in the treatment area, e.g., several days to a few weeks.

### Manufacturing Uniform Components for Use in Integrated Devices of the Disclosure

The disclosure further relates to methods for manufacturing uniform components for use in the integrated devices of the disclosure. In order to generate substantially planar micrografts, the components within cutter assembly 1202 must be substantially uniform with respect to one another. In particular, the planar surfaces of the components within cutter assembly 1202 must be substantially uniform.

In certain aspects one or more coupling members are used to create a frangible coupling between at least two of plate members 1202a, 1202b and 1202c. The coupling members are disposed between two or more of the plate members to form a frangible section that is broken upon movement of said plates with respect to each other, as previously described. The tolerance for any inconsistencies between the planar surfaces of the coupling members and one or more of the plate members and/or inconsistent dimensions (e.g., width) between the coupling members and one or more of the plate members is very low and could result in non-planar, non-uniform micrografts and device malfunction.

Inconsistencies between the planar surfaces of different stocks of sheet material, manufacturing methods of blanks for the coupling members and/or plates, and finishing methods of the coupling members and/or plates can each increase tolerance stackups beyond an acceptable level, thereby decreasing the efficiency and function of device and resulting in micrografts that are unusable, and increase patient discomfort/distress.

The accumulated variations in production dimensions of the coupling members, variations in production dimensions of the plate members, and variation in the spacing between plate members, can each increase tolerance stackups and decrease device function. In order to optimize the tolerances within the cutter assembly **1202,** the plurality of coupling members are preferably formed from the same sheet stock of material as at least one plate member in the cutter assembly **1202.** In a particular embodiment, the plurality of coupling members and at least middle plate member **1202b** in cutter assembly **1202** are preferably formed from the same sheet stock of material (e.g., a single sheet stock of material). Forming the coupling members and the middle plate member **1202b** from the same sheet stock ensures a uniform thickness between the coupling members and between the coupling members and plate member **1202b,** and ensures uniform, planar mating surfaces between the coupling members and plate member **1202b,** thereby decreasing tolerance stackups within cutter assembly **1202** and ensuring proper device function.

Plate members **1202a, 1202b,** and **1202c** can be formed from the same material, or different materials with respect to each other, so long as the materials used result in substantially planar mating surfaces between the three plates. Preferably, plate members **1202a, 1202b,** and **1202c** are formed from a metallic material (e.g., the same metallic material, or different metallic materials).

In certain embodiments, each of plate member **1202a, 1202b,** and **1202c** is formed from the same sheet stock of material, preferably a single sheet stock of material. One or more openings (e.g., holes or slots) are formed within each plate member to form hole arrays **1214** that align when the plate members are assembled, as previously described. In certain embodiments, the coupling members are formed from the same sheet stock from which the plurality of plate members are generated. Forming the coupling members and plate members from the same sheet stock ensures uniformity in the thickness among and between the coupling members and plate members, and uniformly planar mating surfaces between the coupling members and plate members, thereby decreasing tolerance stackups within cutter assembly **1202** and ensuring proper device function.

The coupling members can be any shape or dimension sufficient to couple the plates together without obstructing the holes in the hole arrays **1214** through which the suction blisters are raised. For example, the coupling members can be substantially square or rectangular in shape. Alternatively, the coupling members are substantially circular in shape. In certain embodiments, the coupling members are of a sufficient shape and size for location between the holes of the hole arrays **1214** of the plate members. In other embodiments, the coupling members are of a sufficient shape and size for location along the edges of the plurality of plates.

Any method can be used to manufacture the plates and/or coupling members, such as drilling, milling, laser etching, lithographic processing, photo etching, laser ablation and the like. In a particular embodiment, a photo etching process is used to manufacture the plates and/or coupling members.

The frangible coupling between the coupling members and plate members can be accomplished using a variety of techniques. For example, the coupling members can be frangibly coupled between the plate members via spot welding techniques (e.g., laser spot welding), via an adhesive such as epoxy, polyurethane, acrylic or a resin, via a frangible pin, a snap-fit or tongue and groove assembly. Such frangible coupling techniques can be accomplished using one or more manufacturing processes such as cold-heading, multiple-die forming, multiple-die progression, multiple-die headers, casting, stamping, punching, atomic hydrogen welding, bare metal arc welding, carbon arc welding, flux cored arc welding, gas metal arc welding, gas tungsten arc welding, plasma arc welding, shielded metal arc welding, submerged arc welding, air acetylene welding, oxyacetylene welding, oxygen/propane welding, oxy hydrogen welding, pressure gas welding, resistance spot welding, resistance seam welding, projection welding, flash welding, upset welding, coextrusion welding, cold pressure welding, diffusion welding, explosion welding, electromagnetic pulse welding, forge welding, friction welding, friction stir welding, hot pressure welding, hot isostatic pressure welding, roll welding, ultrasonic welding, electron beam welding, electroslag welding, flow welding, induction welding, laser beam welding, percussion welding, thermite welding, electrogas welding, and stud arc welding.

Optionally, a portion of the plate material at or around the site of the frangible coupling is removed to accommodate at least a portion of the coupling member by forming a depression at or around the frangible section. For example, in one embodiment, laser etching or photo etching on the plate member is used to circumscribe the coupling point at or proximal to the frangible coupling. In another embodiment, a depression at or proximal to the plate member can be removed with any method known in the art, for example drilling, milling, laser etching, photo etching, laser ablation and the like.

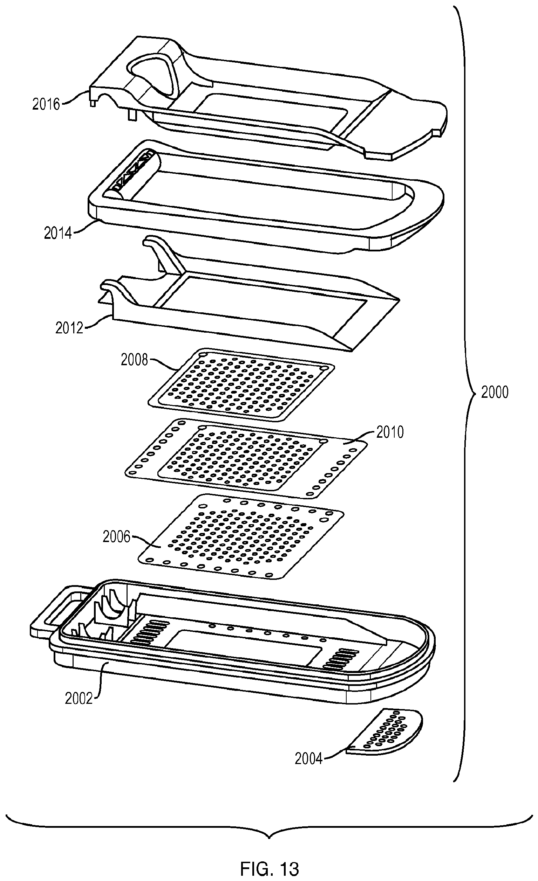

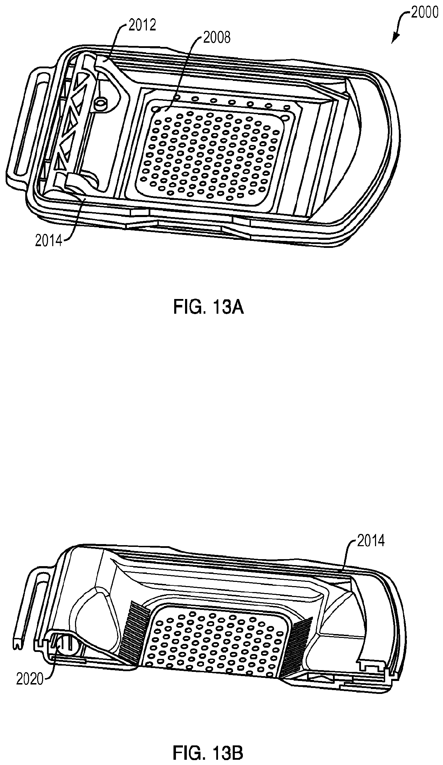

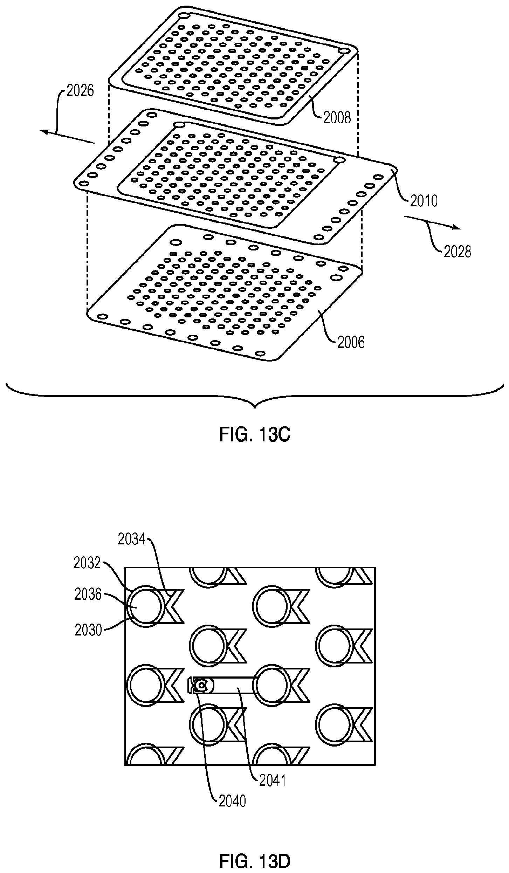

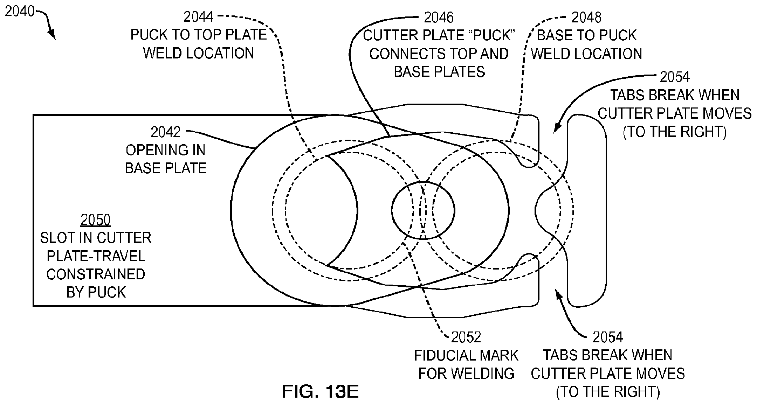

Figure 13 is exploded view of another embodiment of a skin harvester according to the invention. Harvester **2000** includes a bottom element **2002** with a strap coupler **2004** (e.g., for joining a hook and fastener-type strap to the harvester to facilitate attachment of the harvester **2000** to a patient's skin, e.g., by wrapping the device around a patient's leg for harvesting skin from the inner thigh).

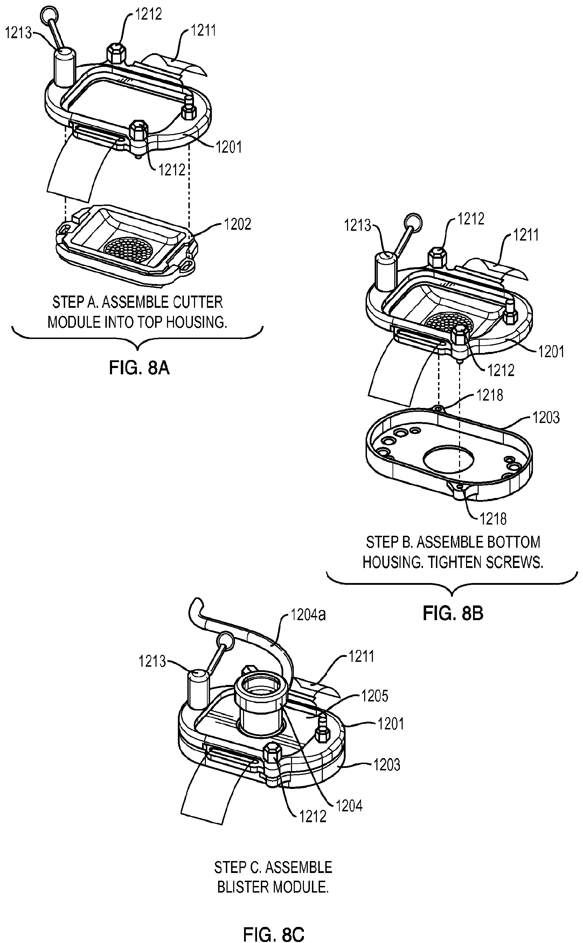

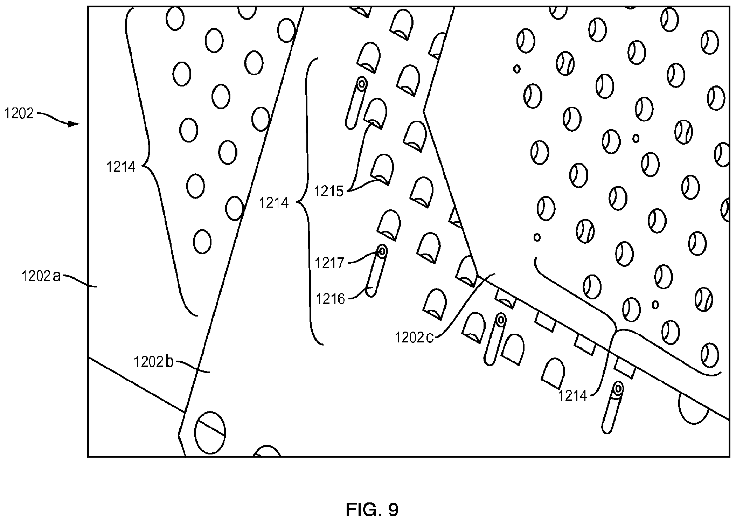

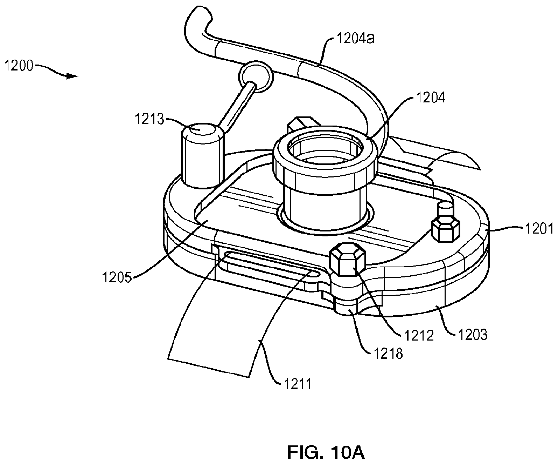

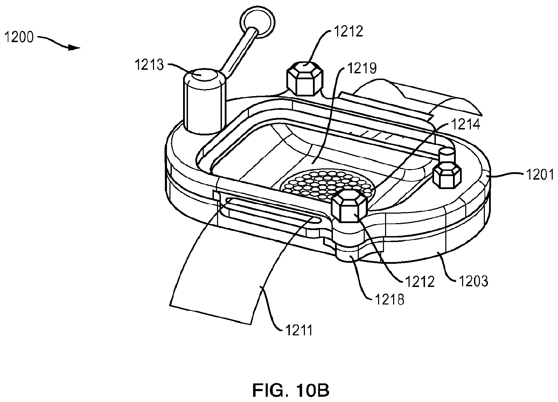

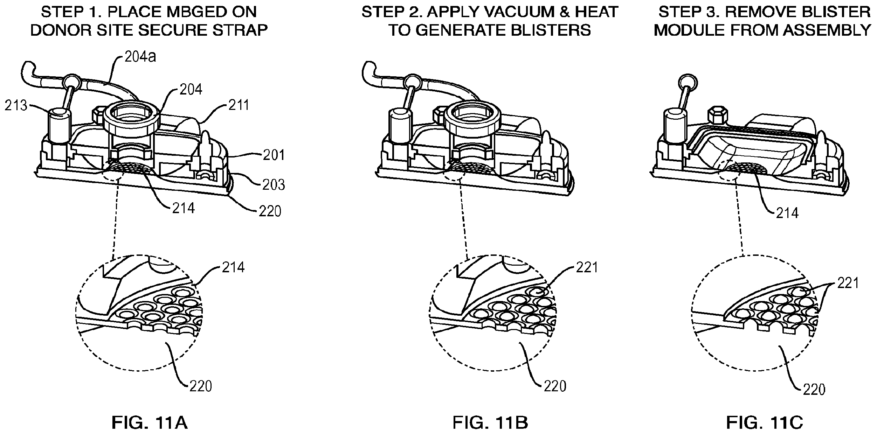

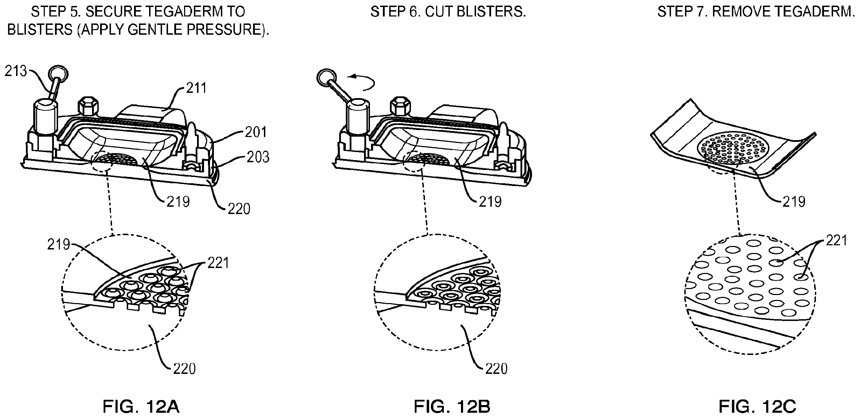

The harvester **2000** also includes a cutter assembly with a bottom plate **2006,** a top plate **2008** and a middle (cutter) plate **2010** configurable to initially provide concentrically aligned holes through which blisters can be raised. (The operation of the plates, **2006, 2008** and **2010** of the cutter assembly is similar to that of elements **1202a, 1202b** and **1202c** described above in connection with Figures 7-12.) The harvester **2000** further includes a cutter drive sled **2012,** handle actuator **2014** and a top element **2016.**

**Figure** 13A is a schematic perspective view of the harvester **2000** as assembled (with top element 2016 omitted for clarity). Figure 13B is a schematic perspective sectional view of the harvester **2000.**

Figure 13C is an exploded schematic perspective view of the guide and cutter plates **2006, 2008** and **2010** of the harvester **2000.** As described above in Figures 7-12, the cutter plate **2010** is designed to move a direction parallel to guide plates **2006** and **2008** in a one-time, back and forth motion. Following the formation of microblisters that protrude through the aligned holes, the cutter plate moves in the direction **2026** to slice the blisters and then moved in the opposite direction **2028** to at least partially retract the blade elements. The alignment of the plates is further illustrated in Figure 13D, which is a top view of the cutter and guide plates in a configuration where the top holes **2032,** bottom holes **2030** and cutter plate holes **2036** are aligned to permit blisters to form and pass through all three plates (The holes of the top plate can be larger than those of the bottom plate to facilitate blister formation and/or growth of the blister.) The cutter plate holes, however, also provide a cutting edge **2034** (shown in phantom) to occlude the passageway and cleave the blister when the plate **2010** is moved relative to the top and bottom guide plates **2006, 2008.** Figure 13D further illustrate one of a plurality of plate connecting posts **2040** that join the top and bottom plates together and an alignment slot **2041** which allows the cutter plate to move relative to the stationary top and bottom plates **2006, 2008.**

The various features of the cutting plate can be formed, for example, lithographically by depositing a resist and patterning it (e.g., by expose to light) such that portions of an initial plate blank are protected from etching while other portion can be removed by etching (e.g., to form the holes and alignment slots). The resist can also be patterned to provide a limited amount of protection to the cutting edge portions, thereby shaping them to have less thickness (e.g., like a knife edge). The sharpness of the cutting edges can be further enhanced by electro-polishing which will reduce the overall thickness of the cutting plate.

Figure 13E (taken in conjunction with Figures 13C and 13D) illustrate one way to provide a plate connector assembly. The vertical posts **2040** can be formed by spot welding the top, bottom and cutter plates together. However, the portion of the cutter plate ("puck" **2046)** that joins the top and bottom plates in the weld is designed to break away from the cutter plate **2010,** e.g., at the initiation of the cutting stroke.

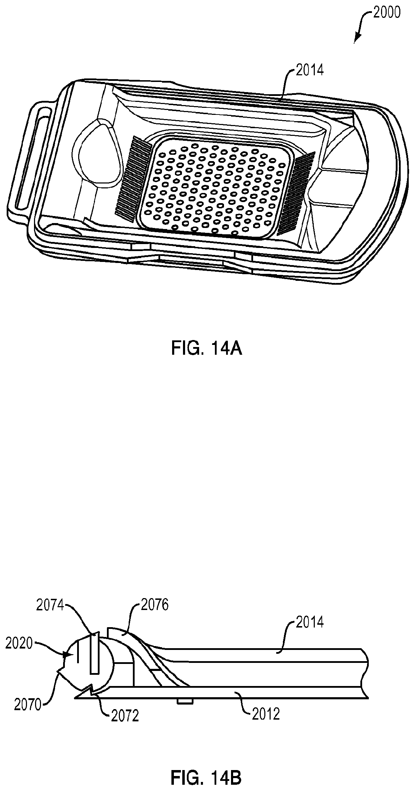

The actuator for moving the cutter plate will now be described in connection with Figures 14-18. Figure 14A is a perspective view of the harvester of Figure 13 in an initial position with the handle (actuator) **2014** in an initial position. The cutter plate **2010** is joined to a sled **2012** as discussed above. The handle is linked to the sled **2012** via a generally cylindrical bar (axle) **2020.** As shown in Figure 14B the cylindrical bar **2020** rotates about an axis when the handle is lifted up or closed. Cylindrical bar **2020,** however also has two non-symmetric features: protrusions **2070** and **2074.** The handle **2014** thus serves as a lever arm.

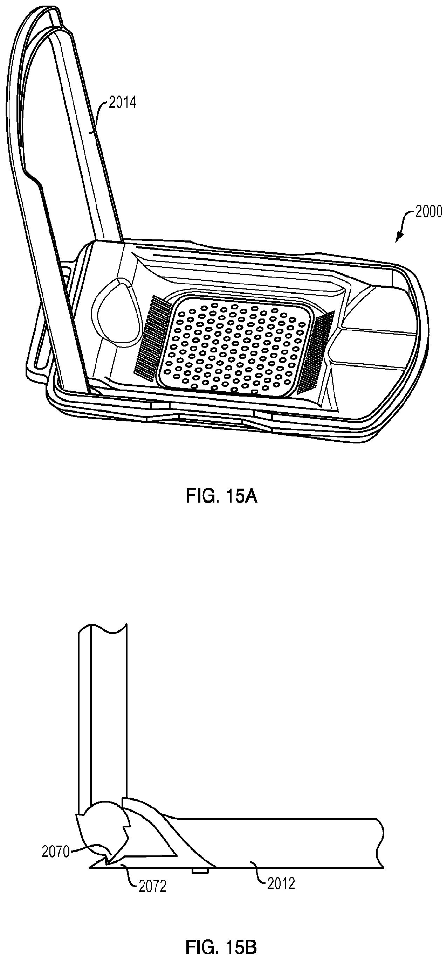

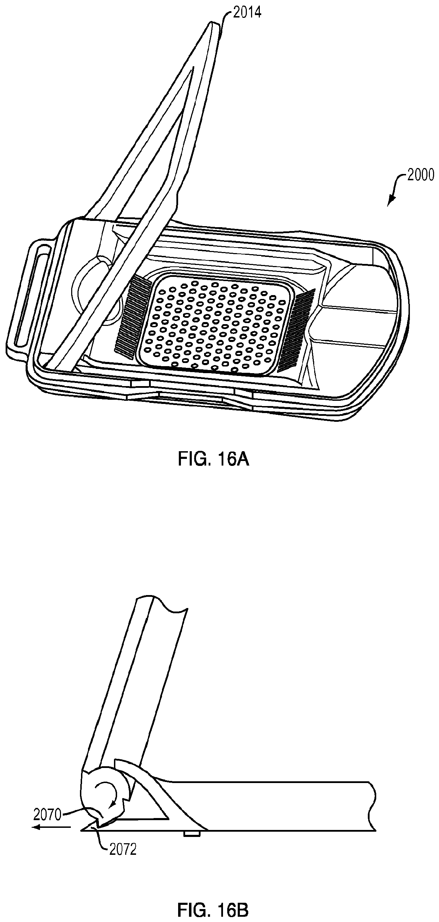

Figure 15A is a perspective view of the harvester in a cocked position (handle up) to further illustrate the cutting mechanism. As shown in Figure 15B this results in protrusion **2070** (e.g., a longitudinal ridge on the cylindrical axle) engaging with a mating feature **2072** (e.g., a groove in the sled). As the handle is brought down the sled is thus forces to move by the rotation of axle **2020.** Figures 16A and 16B illustrates the handle in a in a mid-cut position

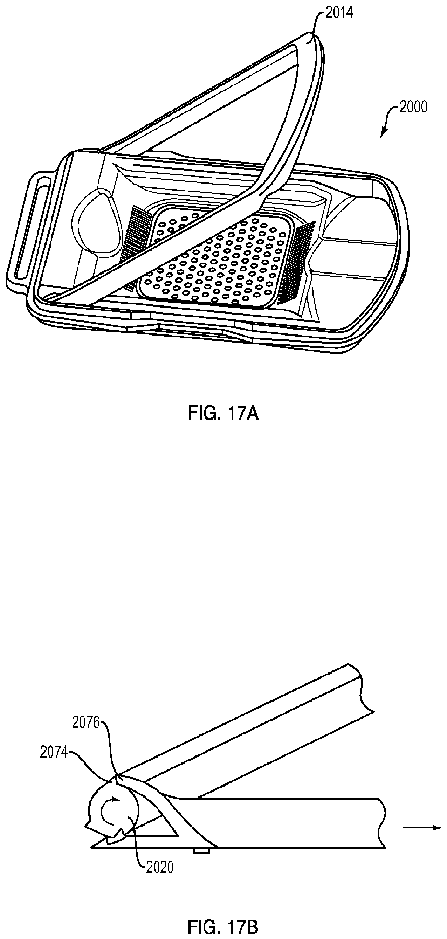

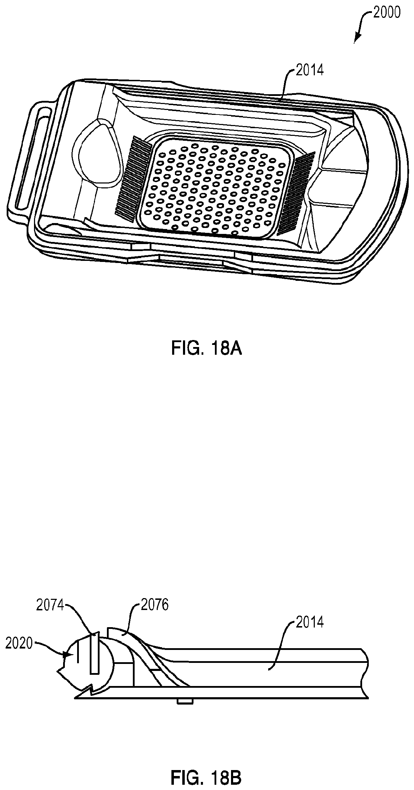

Figure 17A is a perspective view of the harvester of Figure 13 in transitional position (from cutting to retraction) to further illustrate the cutting mechanism. At this point in the rotation of the axle 2020, protrusion 2070 has detached from mating feature 2072 and the second protrusion 2074 has engaged with a different portion 2076 (e.g., a shoulder pad) of the sled. As the handle continues its downward portion, the rotation of axle causes the sled to move in the opposition direct and, thus retract the cutter plate. Figures 18A and 18B illustrate the harvester in a final (stroke completion) position.

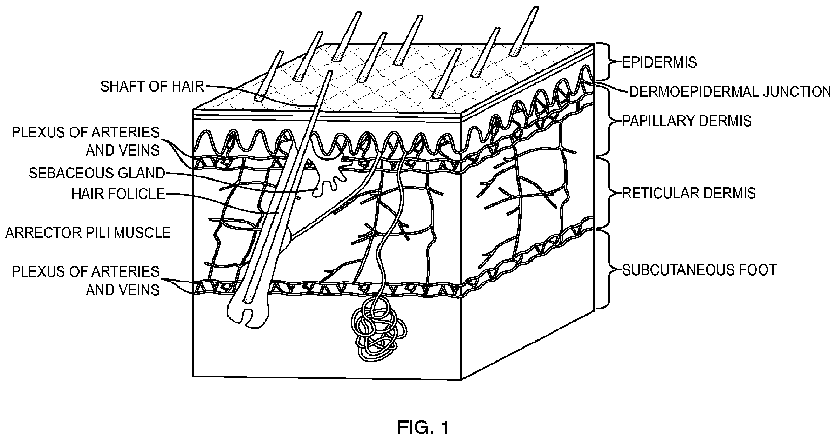

In certain embodiments, devices of the invention are configured to produce epidermal grafts. The skin consists of 2 layers. The outer layer, or epidermis, is derived from ectoderm, and the thicker inner layer, or dermis, is derived from mesoderm. The epidermis constitutes about 5% of the skin, and the remaining 95% is dermis. Figure 1 provides a diagram showing the anatomy of skin. The skin varies in thickness depending on anatomic location, gender, and age of the individual. The epidermis, the more external of the two layers, is a stratified squamous epithelium consisting primarily of melanocytes and keratinocytes in progressive stages of differentiation from deeper to more superficial layers. The epidermis has no blood vessels; thus, it must receive nutrients by diffusion from the underlying dermis through the basement membrane, which separates the 2 layers.

The dermis is a more complex structure. It is composed of 2 layers, the more superficial papillary dermis and the deeper reticular dermis. The papillary dermis is thinner, including loose connective tissue that contains capillaries, elastic fibers, reticular fibers, and some collagen. The reticular dermis includes a thicker layer of dense connective tissue containing larger blood vessels, closely interlaced elastic fibers, and coarse, branching collagen fibers arranged in layers parallel to the surface. The reticular layer also contains fibroblasts, mast cells, nerve endings, lymphatics, and some epidermal appendages. Surrounding the components of the dermis is the gel-like ground substance composed of mucopolysaccharides (primarily hyaluronic acid), chondroitin sulfates, and glycoproteins.

In a graft, the characteristics of the donor site are more likely to be maintained after grafting to a recipient site as a function of the thickness of the dermal component of the graft. However, thicker grafts require more favorable conditions for survival due to the requirement for increased revascularization. It has been discovered, however, that a substantially epidermal graft according to the disclosure is more likely to adapt to the characteristics of the recipient site.

An epidermal graft refers to a graft that consists of substantially epidermal skin and does not include any substantial portion of the dermal layer. A split thickness graft refers to a graft that includes sheets of superficial (epithelial) and some deep layers (dermal) of skin. A full-thickness graft refers to a graft that includes all of the layers of the skin including blood vessels.

Devices of the disclosure may be used to harvest a skin graft(s) for repair of numerous different types of skin damage. For example, harvested grafts may be used to treat burns (e.g., both thermal and chemical burns), blistering, dermatological conditions (e.g., epidermolysis bullosa or pyoderma gangrenosum), radiation therapy ulcers, diabetic ulcers, ischemic ulcers, trophic ulcers, trauma, or depigmentation (e.g., vitiligo).

In particular embodiments, the skin graft(s) are used to treat vitiligo. Vitiligo is a chronic disorder that causes depigmentation of patches of skin. It occurs when melanocytes, the cells responsible for skin pigmentation, die or are unable to function. Although patches are initially small, they often enlarge and change shape. When skin lesions occur, they are most prominent on the face, hands and wrists. Some lesions have hyper-pigmentation around the edges. Depigmentation is particularly noticeable around body orifices, such as the mouth, eyes, nostrils, genitalia and umbilicus.

Vitiligo is generally classified into two categories, non-segmental vitiligo and Segmental vitiligo. In non-segmental vitiligo (NSV), there is usually some form of symmetry in the location of the patches of depigmentation. New patches also appear over time and can be generalized over large portions of the body or localized to a particular area. Vitiligo where little pigmented skin remains is referred to as *vitiligo universalis*. Non-segmental vitiligo can come about at any age, unlike segmental vitiligo which is far more prevalent in teenage years.

Segmental vitiligo (SV) differs in appearance, aetiology and prevalence from associated illnesses. Its treatment is different from that of non-segmental vitiligo. It tends to affect areas of skin that are associated with dorsal roots from the spine. It spreads much more rapidly than non-segmental vitiligo and, without treatment, it is much more stable/ static in course and not associated with auto-immune diseases.

To treat vitiligo, an autograft is provided to the site of depigmented skin. The graft includes melanocytes, and thus upon the recipient site accepting the graft, the graft will produce pigmented skin at the recipient site. A donor site of pigmented skin is aseptically cleaned prior to harvesting of a skin graft. Standard methods are used to clean the donor site. A typical donor site is an inner thigh, but any area of pigmented skin may be used.

After cleaning, a skin grafted is harvested using devices of the invention. Devices described herein raise and cut a blister(s), such as a suction blister. The area of depigmented skin (i.e., the recipient site), is prepared through aseptic cleaning and dermabrasion. The graft(s) is applied to the dermabraded recipient site. The donor site and the recipient site are dressed and wound care is provided.

## Claims

1. A device for obtaining a skin graft, the device comprising:
a harvester (2000) configured for placement on a target region of a patient's skin and further adapted to form a sealing engagement with a head (600) that provides negative pressure to the target region such that the target region of skin is embraced within an evacuated chamber, the harvester (2000) further comprising:
at least one alignment plate (2008 and 2006) having a plurality of holes (2032 and 2030) through which skin blisters can be raised in the presence of negative pressure; and
a cutting plate (2010) having at least one cutting surface for cleaving skin blisters after they are formed within the chamber;
wherein the cutting plate (2010) includes a plurality of holes (2036) suitable for concentric alignment with holes (2032 and 2030) in the alignment plate (2008 and 2006) in the first position to facilitate blister formation and a plurality of cutting surfaces suitable for cleaving blister in the second position,
wherein the device further comprises an actuator (2014) for moving the cutting plate (2010) from the first position to the second position, **characterized in that**
a single stroke of the actuator (2014) moves the cutting plate (2010) from the first position to the second position so as to cleave the skin blister and from the second position to the first position to at least partially retract the cutting plate (2010).

2. The device according to claim 1, wherein said at least one alignment plate (2008 and 2006) comprises a top alignment plate (2008) and a bottom alignment plate (2006) and wherein the cutting plate (2010) is disposed therebetween.

3. The device according to claim 2, wherein the top (2008) and bottom (2006) alignment plates are joined together by a plurality of vertical posts that pass through slots in the cutting plate (2010) to maintain the fixed position of the top (2008) and bottom (2006) plates relative to each other while permitting movement of cutting plate (2010).

4. The device according to claim 2, wherein the top plate (2008) comprises a radiation absorbing material.

5. The device according to claim 2, wherein the top plate (2008) comprises at least one fluoropolymer surface.

6. The device according to claim 2, wherein the top plate (2008), bottom plate (2006) and cutting plate (2010) each have a plurality of holes (2032 and 2030) that are adapted to be concentrically aligned to facilitate blister formation.

7. The device according to claim 6, wherein the holes (2032) of the top plate (2008) are larger than the holes (2030) of the bottom plate (2006).

8. The device according to claim 1, wherein the cutting plate (2010) and the alignment plate (2008 and 2006) are coupled in the first position to prevent relative movement thereof, and wherein actuation of the actuator (2014) is configured to decouple the cutting plate (2010) and the alignment plate (2008 and 2006).

9. The device according to claim 8, wherein the cutting plate (2010) and the alignment plate (2008 and 2006) are frangibly coupled via one or more spot welds that are configured to break upon actuation of the actuator (2014).

10. The device according to claim 1, further comprising a sled (2012) coupled to the cutting plate (2010), and wherein the actuator (2014) comprises a handle (2014) and an axle (2020), the axle (2020) having a first protrusion (2070) extending therefrom for engaging a first mating feature (2072) on the sled (2012) so as to move the cutting plate (2010) from the first position to the second position during a first portion of the rotation of the actuator (2014), and wherein a second protrusion (2074) extending from the axle (2020) engages a second mating feature (2076) on the sled (2012) so as to move the cutting plate (2010) from the second position to the first position during a second portion of the rotation of the actuator (2014).

11. The device according to claim 1, wherein each of the cutting surfaces is associated with one of the holes (2036) of the cutting plate (2010).

12. The device according to claim 1, wherein the head (600) further comprises at least one temperature measuring element for measuring the temperature of the skin or evacuated chamber, a sealing surface to engage with a mating surface on the harvester (2000) such that when the head (600) is engaged with the harvester (2000) on a patient's skin the evacuated chamber is formed over the target region of skin, or a combination thereof.

## Patentansprüche

1. Vorrichtung zur Gewinnung eines Hauttransplantats, wobei die Vorrichtung umfasst:
einen Harvester (2000), ausgelegt zum Platzieren auf einer Zielregion einer Haut eines Patienten und weiter angepasst zur Bildung eines abdichtenden Eingriffs mit einem Kopf (600), der negativen Druck für die Zielregion bereitstellt, sodass die Zielregion der Haut in einer evakuierten Kammer umgeben ist, wobei der Harvester (2000) weiter umfasst:
mindestens eine Ausrichtungsplatte (2008 und 2006), die eine Vielzahl von Öffnungen (2032 und 2030) aufweist, durch die Hautblister in Gegenwart von negativem Druck angehoben werden können; und
eine Schneidplatte (2010), die mindestens eine Schneidfläche zum Spalten von Hautblistern, nachdem sie in der Kammer gebildet worden sind, aufweist;
wobei die Schneidplatte (2010) eine Vielzahl von Öffnungen (2036), die zur konzentrischen Ausrichtung mit Öffnungen (2032 und 2030) in der Ausrichtungsplatte (2008 und 2006) in der ersten Position zum Ermöglichen von Blisterbildung geeignet sind und eine Vielzahl von Schneidflächen, die zum Spalten von Blistern in der zweiten Position geeignet sind, enthält,
wobei die Vorrichtung weiter einen Betätiger (2014) zum Bewegen der Schneidplatte (2010) von der ersten Position zu der zweiten Position umfasst, **dadurch gekennzeichnet, dass**
ein einziger Takt des Betätigers (2014) die Schneidplatte (2010) von der ersten Position zu der zweiten Position, sodass die Hautblister abspalten und von der zweiten Position zu der ersten Position, um mindestens teilweise die Schneidplatte (2010) zurückzuziehen, bewegt.

2. Vorrichtung nach Anspruch 1, wobei die mindestens eine Ausrichtungsplatte (2008 und 2006) eine obere Ausrichtungsplatte (2008) und eine untere Ausrichtungsplatte (2006) umfasst und wobei die Schneidplatte (2010) dazwischen angeordnet ist.

3. Vorrichtung nach Anspruch 2, wobei die oberen (2008) und unteren (2006) Ausrichtungsplatten durch eine Vielzahl von vertikalen Pfosten miteinander verbunden sind, die durch Schlitze in der Schneidplatte (2010) führen, zum Aufrechterhalten der fixierten Position der oberen (2008) und unteren (2006) Platten bezogen zueinander, während eine Bewegung von Schneidplatte (2010) erlaubt ist.

4. Vorrichtung nach Anspruch 2, wobei die obere Platte (2008) ein strahlungsabsorbierendes Material umfasst.

5. Vorrichtung nach Anspruch 2, wobei die obere Platte (2008) mindestens eine Fluorpolymeroberfläche umfasst.

6. Vorrichtung nach Anspruch 2, wobei die obere Platte (2008), untere Platte (2006) und Schneidplatte (2010) jede eine Vielzahl von Öffnungen (2032 und 2030) aufweisen, die angepasst sind, um zum Ermöglichen von Blisterbildung konzentrisch ausgerichtet zu sein.

7. Vorrichtung nach Anspruch 6, wobei die Öffnungen (2032) der oberen Platte (2008) größer als die Öffnungen (2030) der unteren Platte (2006) sind.

8. Vorrichtung nach Anspruch 1, wobei die Schneidplatte (2010) und die Ausrichtungsplatte (2008 und 2006) in der ersten Position zum Verhindern einer relativen Bewegung davon gekoppelt sind, und wobei eine Betätigung des Betätigers (2014) zum Entkoppeln der Schneidplatte (2010) und der Ausrichtungsplatte (2008 und 2006) ausgelegt ist.

9. Vorrichtung nach Anspruch 8, wobei die Schneidplatte (2010) und die Ausrichtungsplatte (2008 und 2006) über eine oder mehrere Punktschweißungen zerbrechlich gekoppelt sind, die zum Brechen nach Betätigung des Betätigers (2014) ausgelegt sind.

10. Vorrichtung nach Anspruch 1, weiter umfassend einen Schlitten (2012), gekoppelt an die Schneidplatte (2010), und wobei der Betätiger (2014) einen Handgriff (2014) und eine Achse (2020) umfasst, wobei die Achse (2020) einen ersten Vorsprung (2070) aufweist, der sich daraus zum Eingriff in ein erstes zusammenpassendes Merkmal (2072) an dem Schlitten (2012) erstreckt, sodass sich die Schneidplatte (2010) von der ersten Position zu der zweiten Position während eines ersten Abschnitts der Rotation des Betätigers (2014) bewegt, und wobei ein zweiter Vorsprung (2074), der sich von der Achse (2020) erstreckt, in ein zweites zusammenpassendes Merkmal (2076) an dem Schlitten (2012) eingreift, sodass sich die Schneidplatte (2010) von der zweiten Position zu der ersten Position während eines zweiten Abschnitts der Rotation des Betätigers (2014) bewegt.

11. Vorrichtung nach Anspruch 1, wobei jede der Schneidflächen zu einer der Öffnungen (2036) der Schneidplatte (2010) zugehörig ist.

12. Vorrichtung nach Anspruch 1, wobei der Kopf (600) weiter mindestens ein Temperaturmesselement zum Messen der Temperatur der Haut oder der evakuierten Kammer, eine abdichtende Oberfläche zum Eingriff mit einer zusammenpassenden Oberfläche an dem Harvester (2000) umfasst, sodass wenn der Kopf (600) im Eingriff mit dem Harvester (2000) auf einer Haut eines Patienten ist, die evakuierte Kammer über der Zielregion der Haut gebildet wird, oder eine Kombination davon.

## Revendications

1. Dispositif pour obtenir une greffe cutanée, le dispositif comprenant :
un dispositif de prélèvement (2000) configuré pour être placé sur une région cible de la peau d'un patient et en outre adapté à former un engagement étanche avec une tête (600) qui fournit une pression négative à la région cible de sorte que la région cible de la peau est englobée dans une chambre sous vide, le dispositif de prélèvement (2000) comprenant en outre :
au moins une plaque d'alignement (2008 et 2006) ayant une pluralité d'orifices (2032 et 2030) à travers lesquels des cloques cutanées peuvent être soulevées en présence de pression négative ; et
une plaque de coupe (2010) ayant au moins une surface de coupe pour détacher des cloques de peau après leur formation dans la chambre ;
dans lequel la plaque de coupe (2010) inclut une pluralité d'orifices (2036) adaptés à un alignement concentrique avec des orifices (2032 et 2030) dans la plaque d'alignement (2008 et 2006) dans la première position pour faciliter la formation de cloques et une pluralité de surfaces de coupe adaptées à détacher une cloque dans la seconde position ;
dans lequel le dispositif comprend en outre un actionneur (2014) pour déplacer la plaque de coupe (2010) de la première position à la seconde position, **caractérisé en ce que**
une seule course de l'actionneur (2014) déplace la plaque de coupe (2010) de la première position à la seconde position afin de détacher la cloque cutanée et de la seconde position à la première position pour rétracter au moins partiellement la plaque de coupe (2010).

2. Dispositif selon la revendication 1, dans lequel ladite au moins une plaque d'alignement (2008 et 2006) comprend une plaque d'alignement supérieure (2008) et une plaque d'alignement inférieure (2006) et dans lequel la plaque de coupe (2010) est disposée entre elles.

3. Dispositif selon la revendication 2, dans lequel les plaques d'alignement supérieure (2008) et inférieure (2006) sont réunies par une pluralité de montants verticaux qui traversent des fentes dans la plaque de coupe (2010) pour maintenir fixe la position des plaques supérieure (2008) et inférieure (2006) l'une par rapport à l'autre tout en permettant le mouvement de la plaque de coupe (2010).

4. Dispositif selon la revendication 2, dans lequel la plaque supérieure (2008) comprend un matériau absorbant les rayonnements.

5. Dispositif selon la revendication 2, dans lequel la plaque supérieure (2008) comprend au moins une surface en fluoropolymère.

6. Dispositif selon la revendication 2, dans lequel la plaque supérieure (2008), la plaque inférieure (2006) et la plaque de coupe (2010) ont chacune une pluralité d'orifices (2032 et 2030) qui sont adaptés à être alignés concentriquement pour faciliter la formation de cloques.

7. Dispositif selon la revendication 6, dans lequel les orifices (2032) de la plaque supérieure (2008) sont plus grands que les orifices (2030) de la plaque inférieure (2006).

8. Dispositif selon la revendication 1, dans lequel la plaque de coupe (2010) et la plaque d'alignement (2008 et 2006) sont couplées dans la première position pour empêcher leur déplacement relatif, et dans lequel l'actionnement de l'actionneur (2014) est configuré pour désaccoupler la plaque de coupe (2010) et la plaque d'alignement (2008 et 2006).

9. Dispositif selon la revendication 8, dans lequel la plaque de coupe (2010) et la plaque d'alignement (2008 et 2006) sont couplées de manière cassable via une ou plusieurs soudures par points qui sont configurées pour se rompre lors de l'actionnement de l'actionneur (2014).

10. Dispositif selon la revendication 1, comprenant en outre un patin (2012) couplé à la plaque de coupe (2010), et dans lequel l'actionneur (2014) comprend une poignée (2014) et un axe (2020), l'axe (2020) ayant une première saillie (2070) s'étendant depuis celui-ci pour venir en prise avec un premier élément d'accouplement (2072) sur le patin (2012) afin de déplacer la plaque de coupe (2010) de la première position à la seconde position pendant une première partie de la rotation de l'actionneur (2014), et dans lequel une seconde saillie (2074) s'étendant depuis l'axe (2020) engage un second élément d'accouplement (2076) sur le patin (2012) de manière à déplacer la plaque de coupe (2010) de la seconde position à la première position pendant une seconde partie de la rotation de l'actionneur (2014).F

11. Dispositif selon la revendication 1, dans lequel chacune des surfaces de coupe est associée à l'un des orifices (2036) de la plaque de coupe (2010).

12. Dispositif selon la revendication 1, dans lequel la tête (600) comprend en outre au moins un élément de mesure de température pour mesurer la température de la peau ou de la chambre sous vide, une surface d'étanchéité destinée à coopérer avec une surface d'engagement sur le dispositif de prélèvement (2000) de sorte que lorsque la tête (600) est en prise avec le dispositif de prélèvement (2000) sur la peau d'un patient la chambre sous vide est formée au-dessus de la région cible de la peau, ou une combinaison de celles-ci.
